# EUROPEAN PATENT SPECIFICATION

(11) **EP 1 643 444 B1**
(45) Date of publication and mention of the grant of the patent: **13.08.2008**
(21) Application number: 04023437.9
(22) Date of filing: 01.10.2004
(51) Int. Cl.: G06T 7/00

(54) **Registration of a medical ultrasound image with an image data from a 3D-scan, e.g. from Computed Tomography (CT) or Magnetic Resonance Imaging (MR)**
Registrierung eines Ultraschallbildes mit einem Bild aus einem 3D-Scan, beispielsweise von einer Computertomographie (CT) oder Magnetspintomographie (MR)
Recalage d'une image ultra-sonique et des données d'image qui sont scannées par example par tomographie assistée par ordinateur ou imagerie par resonance magnetique

(43) Date of publication of application: 05.04.2006
(73) Proprietor: MedCom Gesellschaft für medizinische Bildverarbeitung mbH, 64283 Darmstadt (DE); Esaote S.p.A., 20123 Milano (IT)
(72) Inventor: Grimm, Marcus, 63329 Egelsbach (DE); Sakas, Georgios, 64285 Darmstadt (DE)
(74) Representative: Brunotte, Joachim Wilhelm Eberhard

(56) References cited:
- US-A1- 2001 007 919
- GOBBI D G ET AL: "Ultrasound/MRI overlay with image warping for neurosurgery" MEDICAL IMAGE COMPUTING AND COMPUTER-ASSISTED INTERVENTION - MICCAI 2000. THIRD INTERNATIONAL CONFERENCE. PROCEEDINGS (LECTURE NOTES IN COMPUTER SCIENCE VOL.1935) SPRINGER-VERLAG BERLIN, GERMANY, 14 October 2000 (2000-10-14), pages 106-114, XP002317964 ISBN: 3-540-41189-5
- LAVALLEE S ET AL: "Building a hybrid patient's model for augmented reality in surgery: A registration problem" COMPUTERS IN BIOLOGY AND MEDICINE, NEW YORK, NY, US, vol. 25, no. 2, March 1995 (1995-03), pages 149-164, XP004532284 ISSN: 0010-4825
- NORTHERN DIGITAL INC.: "The POLARIS Family of Optical Tracking Systems" INTERNET ARTICLE, [Online] 10 July 1998 (1998-07-10), XP002317965 Retrieved from the Internet: URL:http://web.archive.org/web/19980710053 757/ndigital.com/polaris.html> [retrieved on 2005-02-15]
- PAGOULATOS N ET AL: "Interactive 3D registration of ultrasound and magnetic resonance images based on a magnetic position sensor" IEEE TRANSACTIONS ON INFORMATION TECHNOLOGY IN BIOMEDICINE IEEE USA, vol. 3, no. 4, 31 December 1999 (1999-12-31), pages 278-288, XP002317966 ISSN: 1089-7771
- ROCHE A ET AL: "RIGID REGISTRATION OF 3-D ULTRASOUND WITH MR IMAGES: A NEW APPROACH COMBINING INTENSITY AND GRADIENT INFORMATION" IEEE TRANSACTIONS ON MEDICAL IMAGING, IEEE INC. NEW YORK, US, vol. 20, no. 10, October 2001 (2001-10), pages 1038-1049, XP001107728 ISSN: 0278-0062

## Description

The invention relates to the registration of first image data of an object and of second image data of the object. The first image data are generated, or have been generated, by an ultrasound (US) detector. In particular, the US image data are (locally) two-dimensional. The second image data are (locally) three-dimensional image data.

The subject-matter of this application may be combined and/or extended with the subject-matter of United States patent application, serial number 10/815759, filed on April 2, 2004, and with the subject-matter of European patent application, filing number 03008448.7, filed on April 11, 2004. The subject-matter of these two patent applications is herewith incorporated by reference.

To be able to compare images, or to combine images, the contents of the images must be in alignment. If the different image data are generated by different detectors and/or at different times, this is usually not the case. The process of finding the correspondence between the contents of images is called image registration. In other words: registration means finding a geometric link which non-ambiguously links the locations and orientations of the same objects, same parts of objects and/or same structures in different image data. For example, such a link may comprise information which define a geometric transformation of one of the image data so that the transformed image data and the other image data are defined with respect to the same coordinate system.

The second image data may be generated by a computer tomography (CT), a magnetic resonance (MR), a positron emission tomography (PET), an X-ray and/or a three-dimensional (3D) US imaging device. In particular, any (locally) 3D image information can be used as the second image data.

More specifically, the invention relates to the field of combining US image data with the second image data. A combination device may combine the US image data and the second image data of the object. The combined image data may be displayed in separate areas of a screen and/or may be superimposed on a screen. Even more specifically, the invention may be used to support diagnosis and/or treatment concerning human or animal bodies, but also concerning material research and/or material examination.

Ultrasound detectors are comparatively easy to handle and are able to deliver image information quasi-continuously and, approximately, in real-time. However, for many applications, other imaging technologies (such as the ones mentioned above) provide better results. By combining the ultrasound image data and the second image data, it can be simulated that the ultrasound detector generates image data having the higher quality of the second image data. The movement of the ultrasound detector may be tracked and the second image data may be displayed which correspond to the instantaneous position and viewing direction of the ultrasound detector. However, a precise registration of the different image data is necessary for this purpose.

A user of an ultrasound detector may register the ultrasound image data and the second image data manually, e.g. by moving the ultrasound probe relatively to the object until an ultrasound image and an image of the second image data are aligned. However, aligning the images with regard to several degrees of freedom is a complicated procedure. On the other hand, many experienced users prefer to manually register the image data rather than relying on automatic systems. Furthermore, markers may be attached to the object, wherein the markers are visible in both types of image data. The marker positions are aligned during the registration procedure.

David G. Gobbi et al.: "Ultrasound/MRI overlay with image warping for neurosurgery", in: Medical image computing and computer-assisted intervention - Miccai 2000. Third international conference. Proceedings (lecture notes in computer science volume 1935) Springer-Verlag Berlin, Germany, 14 October 2000, pages 106 to 114, XP002317964 ISBN: 3-540-41189-5, describes a system which provides the means for overlaying both 2D ultrasound video and 3D ultrasound image volumes with 3D MRI volumes. An ultrasound probe is tracked by a 3D optical tracking system. 2D and 3D image overlay tools allow interactive, real-time visualisation of a brain tissue shift which may occur between recordation of an MRI 3D image and recordation of an ultrasound image.

S. Lavalle et al.: "Building a hybrid patient's model for augmented reality in surgery: A registration problem", in: Computers in biology and medicine, New York, NY, US, vol. 25, no. 2, March 1995, pages 149 to 164, XP004532284, ISSN: 0010-4825, describes in section 2 existing methods of the registration of 3-D spaces. In section 2.1, the matching of surfaces with surfaces or surface points is described. Typically, one surfaces can be the result of a 3-D segmentation algorithm on 3-D images (CT, MRI,...). The segmented surface will have to be registered with a second data surface which can be a set of surface patches or points acquired with a large variety of sensors. If the chosen reference structure is visible (i.e. in the case of skin surfaces), standard vision sensors can be used, such as 3-D localisers (optical, ultrasound or mechanical). Ultrasound images spatially registered together using 3-D localisers also yield invaluable information about surface points location, according to the document. However, automated segmentation of ultrasound images is still a very difficult problem in the general case.

It is an object of the present invention to provide a method and an apparatus which facilitate the registration of ultrasound image data and of second, three-dimensional image data.

It is proposed to support the (in particular manual) registration of the first and second image data by performing an automatic partial registration. Thus, the user does not have to perform the complete registration on his or her own.

The appended claims define the scope of protection.

The registration with respect to one registration direction may include transforming coordinates of the first and/or second image data so that the positions of the reference location and of the surface point in a joint coordinate system is adapted. In particular, the positions in the one registration direction becomes identical by performing the transformation, if the reference location is located on the surface of the object. Furthermore, the result of the partial registration may be used in a complete registration, for example in order to determine values of a transformation matrix.

The reference information may be generated by the ultrasound detector. In particular, the reference information may be part of the geometry data described below or may be generated using the geometry data (for further details concerning such geometry data reference is made to United States patent application, serial number 10/815759, filed on April 2, 2004, and to European patent application, filing number 03008448.7). Thus, it is preferred to use an ultrasound detector which is capable of generating such reference information or geometry data, wherein the ultrasound detector may directly be connected to a registration support device (e.g. a computer) which is adapted to support the manual registration.

The reference location in the first image data may be a point, e.g. a so-called "near point" which will be located exactly on the surface of the object (for example on the skin of a patient) when the ultrasound image data are generated. The defined distance of the reference location to the surface of the object is zero in this case. In other words: the near point may be located on the surface of an ultrasound probe of the ultrasound detector, wherein the surface of the probe contacts the surface of the object during generation of the ultrasound image data. The ultrasound detector may "know" the location of the near point in the ultrasound image. Alternatively or in addition to the near point, a near line may be defined, which near line is a tangential line to the surface of the object (or may be parallel to the tangential line). However, instead of a near point or near line, another reference location may be defined in the ultrasound image data. For example, the reference location may be located at a known distance to the surface of the object when the ultrasound probe contacts the object.

The at least one surface point on the surface of the object may be identified by a registration support device. It is possible to identify a plurality of the surface points, wherein for example each of the surface points is located in one of different cut planes of the second image data and/or wherein the surface points are located in the same cut plane of the second image data. In particular, the at least one surface point is an intersecting point of a line with the surface of the object, wherein the line extends in a defined viewing direction. The viewing direction is a defined direction of the ultrasound detector when the ultrasound detector views the object during generation of the ultrasound image data. The term "intersecting" includes the case that the line extends at one side of the surface only, i.e. the line ends at the surface.

The surface point may be identified by evaluating second image data along a straight line which extends in a direction of identification. In particular, the direction of identification may be fixed for a given cut plane of the second image data or may be calculated using information about a shape of the surface of the object. The straight line intersects the surface of the object at at least one point. If there is no such intersecting point for a given straight line within the boundaries of an image of the second image data, a signal may be output to the user and the user may adapt the image and/or the image boundaries. If there is more than one intersecting point, one of the intersecting points may be chosen automatically and the partial registration may be performed on the basis of this intersecting point. However, the user may choose one of the other intersecting points. Alternatively, the partial registration may be performed for more than one of the intersecting points and corresponding results may be provided to the user for selecting an appropriate result.

Several straight lines may be parallel to each other and, therefore, have the same direction of identification. In particular when the direction of identification is fixed for a given cut plane of the second image data, intersecting points can be identified for a plurality of the straight lines in advance and/or repeatedly during the process of manual registration performed by the user. Especially when the user amends (for example by moving the ultrasound probe) the alignment of the first and second image data in a direction transverse to the registration direction with automatic registration, the identification can be repeated. Preferably, corresponding results of the repeated automatic registration are displayed automatically, for example by superimposing images of the first and second image data (see below regarding the display of images).

The user may correct a result of the partial automatic registration. For example, a correction might be necessary if the user deforms the surface of the object by pressing the ultrasound probe against the surface. In particular for this purpose, the following preferred embodiment is proposed: a first image and a second image are displayed according to the partial registration with respect to the one registration direction. The first image corresponds to the first image data and the second image represents a part of the second image data. In addition, the reference location is displayed in the first image and the at least one surface point is displayed in the second image. As a result, the user can compare the reference location and the at least one surface point.

The at least one surface point may be identified comprising one or both of the following steps:
- comparing data values of data points with a threshold value;
- evaluating data values of neighbouring data points and identifying a location where the local partial derivative of the data values matches a threshold value or matches or exceeds a threshold value.

In particular, the data values may be greyscale values of the second image data.

According to a preferred embodiment, a first image of the object may be displayed (for example on a computer screen) corresponding to repeatedly generated ultrasound image data and a second image of the object may be displayed corresponding to the second image data, wherein the orientation and/or scaling of at least a part of the object is identical in the first and in the second image. This type of combining the first and second image data and other types of combining may be performed by a combination device.

In particular, a cut plane may be defined and second image data which are located in the cut plane are displayed. Preferably, the first image and the second image are superimposed on a display device, wherein the first image and the second image are displayed according to the partial automatic registration with respect to the one registration direction. This means that the user can see the result of the partial registration and can finalise the registration easily. For example, the user will manually register the different image data with respect to a second registration direction which is perpendicular to the first registration direction. In addition, both the automatic and manual registration can be performed in a second cut plane which may be perpendicular to the first cut plane.

Furthermore, the user may start the registration procedure by selecting the cut plane and by positioning and aligning the ultrasound probe of the ultrasound detector so that the first image (the displayed ultrasound image) is an image in the cut plane. Preferably, the automatic partial registration according to the invention is immediately performed when the user has chosen the cut plane.

A tracking sensor may be combined with (for example attached to) the ultrasound probe of the ultrasound detector and a tracking system may be provided so that a position and an orientation of the ultrasound probe in a global coordinate system may be tracked.

In addition to the ultrasound image data, the ultrasound detector may generate geometry data and may transfer the geometry data to a registration support device for supporting the manual registration. The geometry data may be used to perform the partial registration described above.

The geometry data comprise one or more than one of the following type of information:
a) information concerning at least one spatial dimension of an image unit of the first image data, in particular of a pixel (preferably separately for different directions of a coordinate system);
b) information concerning an image position of at least a part of an image, which is represented by the first image data, relative to a reference point of the ultrasound detector or relative to a reference point or reference object in the ultrasound image. This information is particularly useful, if a user can adjust a zoom factor of the ultrasound image. For example, this information comprises a distance in image units (e.g. pixels). In combination with the scaling information of item a), the distance may be defined in cm or another unit of length;
c) information concerning an orientation of the ultrasound image relative to a reference point or a reference object of the ultrasound detector (in particular an ultrasound probe of the detector). For example, this information may comprise the orientation of at least one axis of a coordinate system of the ultrasound image; and
d) information concerning a region or an area, which is actually covered by an ultrasound image that is represented by the first image data;
e) and optionally: information concerning a detector position of the ultrasound detector relative to a position sensor for determining a location and/or an orientation of the ultrasound detector. Instead of or in addition to a position sensor, a signal source may be coupled to the ultrasound probe, wherein the signal can be evaluated in order to determine the position of the probe. For example, such information may be collected once in advance and may be saved individually for each ultrasound probe, which can be connected to the ultrasound system/device. In this case, it is sufficient during operation to transfer simply an identification signal, which enables to identify the probe that is used. The combination device can select the respective geometry information using the identification information. In a specific embodiment, the information concerning the relative position, which is transferred or saved, may be a calibration matrix.

Preferably, all of these types of information are transferred from the ultrasound detector to the combination device.

If the ultrasound detector comprises a control unit for controlling an image data generation of the ultrasound detector, the control unit may be adapted to generate at least a part of the geometry data. For example, the control unit can adjust a penetration depth of the ultrasound image, using a velocity value of the ultrasound waves in the object, by setting a time limit for detection of US echo signals. In this case, the control unit can calculate the penetration depth and can transfer information about the penetration depth to the combination device. Furthermore, the width of an image recording area of an ultrasound probe may be available to the control unit for control purposes and the control unit can transfer this information to the combination device.

Furthermore, the ultrasound detector, the combination device and (optionally) further parts or units of an imaging system may be integrated in one and the same device. For example, several or all of the units of such a device may be connected to a data bus system for transferring data.

Furthermore, the present invention includes:
- a computer loadable data structure that is adapted to perform the method according to one of the embodiments described in this description while the data structure is being executed on a computer or computer network,
- a computer program, wherein the computer program is adapted to perform the method according to one of the embodiments described in this description while the program is being executed on a computer or computer network,
- a computer program comprising program portions for performing the method according to one of the embodiments described in this description while the computer program is being executed on a computer or on a computer network,
- a computer program comprising program portions according to the preceding item, wherein the program portions are stored on a storage medium readable to a computer,
- a storage medium, wherein a data structure is stored on the storage medium and wherein the data structure is adapted to perform the method according to one of the embodiments described in this description after having been loaded into a main and/or working storage of a computer or of a computer network,
- a computer program product having program code portions, wherein the program code portions can be stored or are stored on a storage medium and wherein the code portions are adapted to perform the method according to one of the embodiments described in this description, if the program code portions are executed on a computer or on a computer network, and/or
- a computer program product loadable into an internal memory of a computer or of a computer network, comprising program code portions for performing steps c) to e) of the method defined above using data according to items a) and b) of the method defined above; alternatively the method may comprise these steps and items and may comprise any additional feature described in this description.

In the following, examples and preferred embodiments are described with reference to the accompanied drawings. However, the invention is not limited to the features described in the following description. The figures of the drawing schematically show:
- Fig. 1: an arrangement comprising an apparatus for combining ultrasound image data with a second type of data, e.g. CT image data;
- Fig. 2: a more detailed view of the ultrasound detector shown in Fig. 1,
- Fig. 3: schematically the content which is shown on a display device,
- Fig. 4: a part of the content of Fig. 3 which is displayed in an area of the display device,
- Fig. 5: a flow-chart of partially registrating and
- Fig. 6: an arrangement with an apparatus for supporting a manual registration.

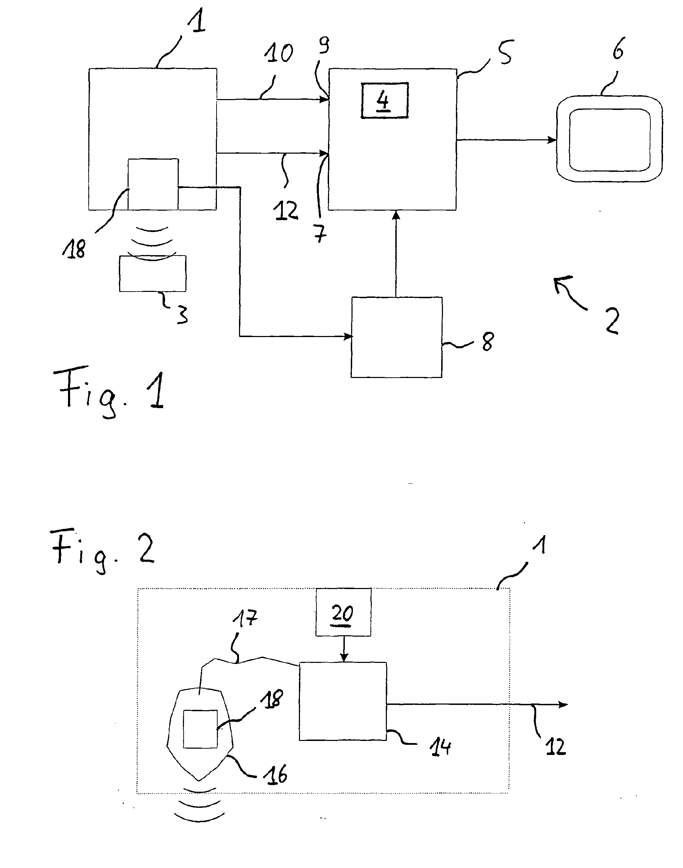

As shown in Fig. 1, an ultrasound detector 1 is connected to a combination device 5 via an image data connection 10. The image data connection 10 is connected to an interface 9 for receiving the ultrasound image data. Images of an object 3 are to be displayed on a display device (for example a screen 6) which is connected to the combination device 5. The combination device 5 may be a computer, such as a personal computer, and may be adapted to perform a partial registration of different image data and/or different images.

The ultrasound detector 1 generates first image data of the object 3 and transfers the first image data to the combination device 5 via the image data connection 10. The combination device 5 comprises a data storage 4 which contains second image data that have previously been generated by a separate device (not shown in Fig. 1). The combination device 5 is adapted to combine the first and second image data and to display them on the screen 6. For example, the first and second image data may be displayed separately on a split screen or may be superimposed. In any case, it is preferred that a first image, which is generated using the first image data, and a second image, which is generated using the second image data, show at least partially the same area or region of the object 3.

The user adjusts the orientation of the ultrasound detector 1 and/or selects an appropriate image from the second image data so that an orientation of the first image and of the second image on the screen 6 is aligned. Furthermore, the user may adjust the geometric scaling (the sizes of image units on the screen 6) of at least one of the images so that the scaling of the first image and of the second image is equal.

The ultrasound detector 1 and the combination device 5 are connected to each other by an additional data connection 12 for transferring geometry data from the ultrasound detector 1 to the combination device 5. The connection 12 is connected to an interface 7 of the combination device 5. In particular, the geometry data connection 12 may be connected (as shown in Fig. 2) to a control unit 14 of the ultrasound detector 1.

In practice, the data connections 10, 12 may be realised by separate data connection links or by the same data connection link. For example, a "link" may comprise a connection line, a plurality of connection lines and/or a digital data bus or bus system.

An ultrasound probe 16 (Fig. 2) of the ultrasound detector 1 is firmly coupled to a position sensor 18 of a tracking system. By determining the orientation and the location of such a position sensor in a global coordinate system (such as the coordinate system of a room) a movement of the ultrasound probe 16 can be tracked. For example, magnetic and/or optical (e.g. infrared) signals may be used by the tracking system. The position sensor 18 is connected to a tracking system control unit 8 and the control unit 8 is connected to the combination device 5. During operation of the arrangement 2, the control unit 8 repeatedly or quasi-continuously transfers information concerning the position and concerning the orientation of the ultrasound probe 16 to the combination unit 5. Alternatively, this information may be transferred directly from the US detector to the combination device. I.e. this information might be at least partially included in the geometry data which are transferred.

As shown in Fig. 2, the ultrasound device 1 may, for example, comprise an ultrasound probe 16, which is connected to the ultrasound control unit 14, for example via a flexible cord 17 for transferring echo signals to the control unit 14. On the other hand, the control unit 14 can transfer control signals to the ultrasound probe via the cord 17. Also, it is possible that at least a part of the geometry information is transferred from the ultrasound probe 16 to the control unit 14 and/or that at least a part of the geometry information generated by the control unit 14 is based on and/or derived from information, which is transferred from the ultrasound probe 16 to the control unit 14.

An input unit 20 is connected to the ultrasound control unit 14, for example for inputting settings of the ultrasound detector, such as a penetration depth and/or range of the ultrasound image. Furthermore, the user may change the orientation of the ultrasound image via the input unit 20.

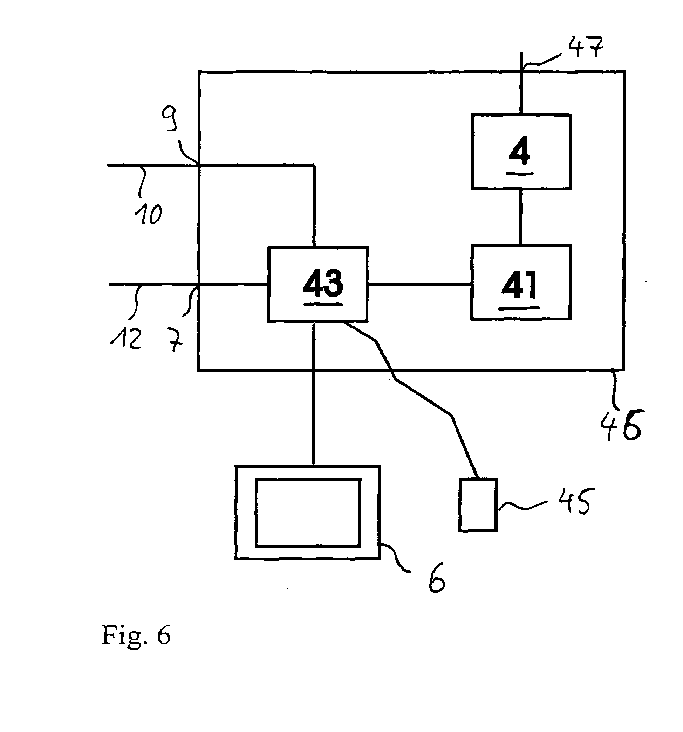

Fig. 6 shows the most preferred embodiment of an apparatus 46 for supporting a manual registration. The apparatus 46 is, for example, a personal computer and is adapted to combine the ultrasound image data with three-dimensional image data (the second image data), such as CT image data. In addition, a user can move an ultrasound probe and/or can input commands to the apparatus 46 so that the apparatus 46 can perform the full registration of the ultrasound data and of the second image data. Because of these user actions (moving the first image and/or inputting commands) the registration is performed "manually", although the apparatus 46 performs the necessary calculations.

The apparatus 46 shown in Fig. 6 may be the combination device 5 of Fig. 1. In addition, the apparatus 46 comprises an interface 47 for inputting the second image data. The interface 47 is connected to the data storage 4. Furthermore, an input device 45 for inputting commands to the apparatus 46 is provided. The input device 45 may comprise a pointer device (such as a trackball or a computer mouse), a keyboard and/or other input means.

The input device 45 is connected to a partial registration device 43. The partial registration device 43 is connected to the interface 7, to the interface 9, to the screen 6 and to an identification device 41 which is connected to the data storage 4. The identification device 41 and/or the partial registration device 43 may be realised by software run on a central processing unit of the apparatus 46 The arrangement shown in Fig. 6 may operate according to the most preferred embodiment of a method for supporting a manual registration of first and second image data, which embodiment is described in the following.

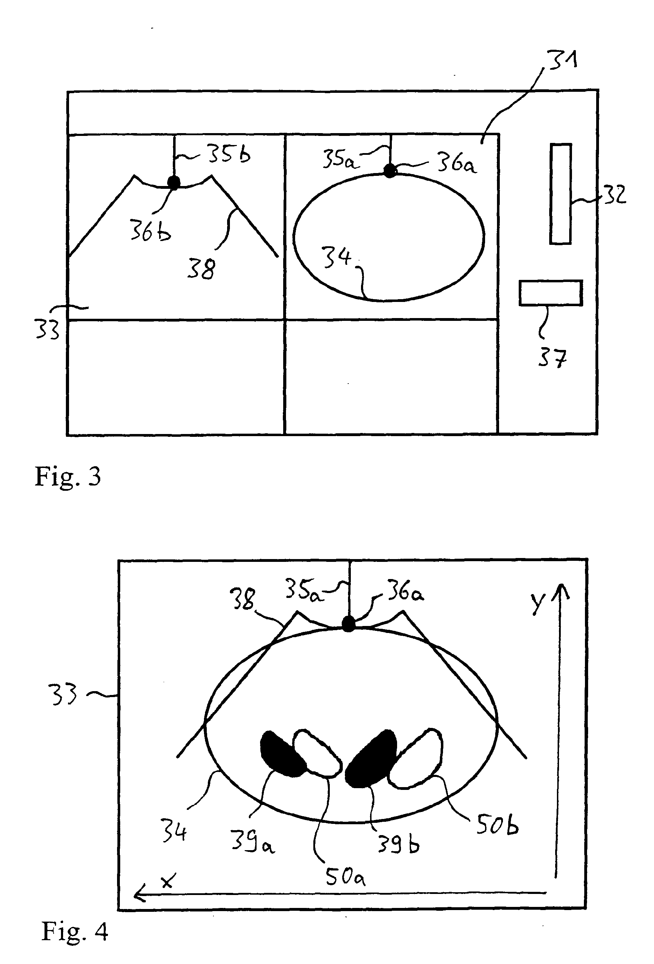

At the beginning of the procedure, the user may choose a slice of the second image data, i.e. he may define and/or select a cut plane and the corresponding second image data may be displayed in an area (e.g. the rectangular area 31 shown in Fig. 3) of a display device (e.g. the screen 6). For example, the user may define that the cut plane is an axial, a sagital or a coronal cut plane of a patient. Furthermore, he may choose a specific cut plane by inputting a command to the apparatus 45. The content of the display device shown in Fig. 3 comprises an area 32 for scrolling through the slices of a defined type of cut planes (the axial cut planes of a patient in the example). In the rectangular area 31, the outline 34 of the body of the patient is schematically shown. The outline 34 is defined by the skin of the patient.

An ultrasonic image is displayed in a second area (e.g. the rectangular area 33 shown in Fig. 3) of the display device. In addition or alternatively, the ultrasonic image and the slice may be superimposed in the same area of the display device. Fig. 4 shows such an area, which may be the rectangular area 33 of Fig. 3. The reference numeral 38 denotes the boundaries of an ultrasonic image area. Within these boundaries 38 only, image data can be collected by the ultrasound detector. The content shown in Fig. 3 comprises further display areas which may be used for other purposes

Alternatively, the user may select an ultrasound image first, may then select the corresponding slice of the second image data and may manually register the ultrasound image and the slice.

The arrangement of the ultrasonic image and of the slice shown in Fig. 3 and Fig. 4 is the result of the partial registration procedure according to the invention. It can be recognised from the area 31 that a vertical line 35a is displayed in the area 31, which line extends from the top boundary of the area 31 to the outline 34 and, thereby, to the surface of the patient (the object). A point 36a marks the location where the line 35a connects the outline 34. This is the "intersecting point" of the straight line 35 with the surface of the object and, more generally speaking, the "surface point" to be identified.

In the preferred embodiment of the invention, a direction of identification is defined for each cut plane. The straight line 35a extends in the defined direction of identification. Furthermore, the straight line 35a may automatically be generated and/or its location may be computed by the apparatus 46, as soon as a slice of the second image data is selected. For example, the straight line 35a is defined as the line which extends in the vertical direction and which cuts the displayed image of the slice in two equal halves. Thus, the straight line 35a is automatically shifted relative to the image data of the slice when the boundaries of the slice are moved in horizontal direction. Furthermore, it is preferred that the corresponding intersecting point 36a is automatically calculated and, optionally, displayed. However, the position of the straight line may be defined differently.

Similarly, a straight line 35b and a point 36b at the lower end of the straight line 35b are shown in the second rectangular area 33 and in Fig. 4. In this example, the straight line 35b is the line which cuts the displayed ultrasonic image in two equal halves and the point 36b is the so-called "near point" (for the definition of the near point see above). The near point is defined by the reference information which is received by the apparatus 46. However, the straight line may be located at other positions.

Although the straight lines 35a, 35b and the points 36a, 36b are displayed in the example of Fig. 3, this is not necessarily the case in other embodiments of the invention.

When the following information is provided:
- the slice of the second image data and its boundaries,
- the direction of identification for identifying the surface point,
- sufficient information in order to define the location of a straight line (such as the straight line 35a),
- the ultrasonic image and
- the reference information (i.e. the information defining the reference location in the ultrasonic image data)
the partial registration procedure automatically calculates the location of the surface point. It is sufficient to calculate the location (e.g. the coordinate of the surface point) with regard to one direction of the second image data, namely with regard to the direction of identification. In the example of Fig. 3 and Fig. 4, the location can be defined by the y-coordinate (see Fig. 4 for the definition of the y-axis).

Alternatively, the surface point on the surface of the object, which corresponds to the near point (or to another reference location) of the ultrasonic image, may be identified in a different manner, in particular without using a direction of identification. For example, the surface outline of the object, or a part of it, may be identified as a line and the surface point may be identified using additional information. This additional information may simply define that the surface point is located half way between the right and left boundary of the displayed image.

When the surface point has been identified, the partial registration procedure is finished by aligning the ultrasonic image and the slice. In the example of Fig. 4, the slice is displayed so that the surface point (point 36a) is located at the same height (the same value of the y-axis) as the near point (point 36b).

Now, the user may complete the registration by moving the ultrasound detector (or by moving the probe of the detector) so that the ultrasonic image is shifted in the horizontal direction (the direction of the x-axis, see Fig. 4). The user may use structures in the superimposed images in order to decide where to move the ultrasound detector. Fig. 4 shows structures 50a, 50b in the second image data and structures 39a, 39b in the ultrasound image data. The structures 50a, 39a and the structures 50b, 39b originate from the same area of the object. Consequently, Fig. 4 shows a situation in which the registration has not been completed yet. When the user has completed the registration, he may click on the button 37 (Fig. 3) in order to inform the apparatus that the registration should be performed on the basis of the instantaneous positions of the two images or on the basis of the instantaneous position of the ultrasound detector.

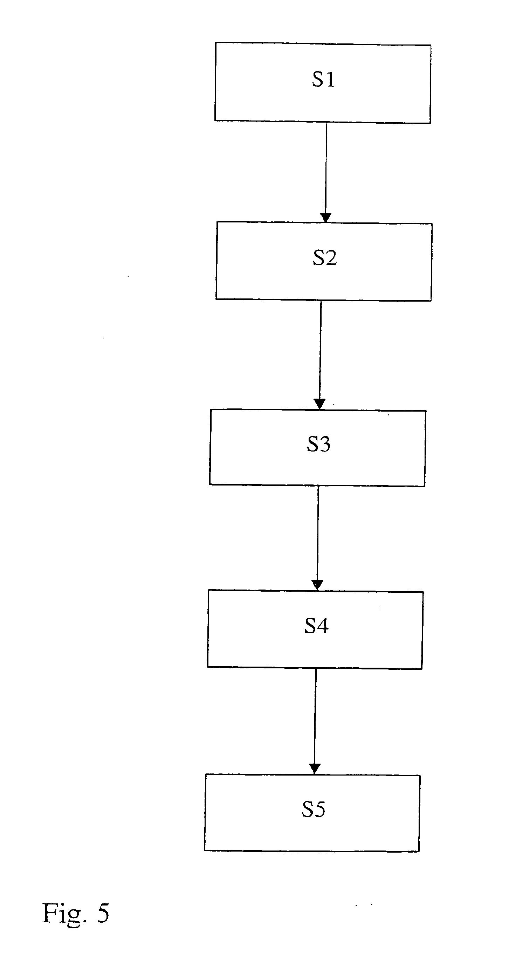

More generally speaking, the automatic partial registration procedure may comprise the steps (Fig. 5):
Step S1: receiving input data, in particular first image data and second image data,
Step S2: receiving reference information which defines a reference location in the first image data,
Step S3: identifying a surface point on the surface of the object,
Step S4: registering the first and second image data with respect to one registration direction and
Step S5: outputting a result of the partial registration.

Steps S1 and S2 may be performed in different order and/or in parallel to each other.

Step S3 may be performed using a software portion comprising the following features:

A starting point in the second image data of a given slice is identified using information about the direction of identification. Then, the values (e.g. greyscale values) of image data points are evaluated in consecutive order in the direction of identification, starting with the starting point. The evaluation is performed until the boundaries of the given slice are reached or until the surface point is identified. For the evaluation, each value of an image data point may be compared with a threshold value. For example, the threshold value is a defined greyscale value which may be chosen so that a skin of a human or animal patient (i.e. the surface of the object) produces significantly higher values and so that an area outside of the patient produces significantly lower values than the threshold value in the second image data (or vice versa). In the case of CT image data, the outside area appears dark and the skin appears bright. Thus, if a starting point in the outside area is identified, the procedure will stop as soon as the first data point of the skin (the surface point) is reached. At least one coordinate of this data point may be returned to the main program and may be used to perform the partial registration.

## Claims

1. A method of automatically performing a partial registration of first image data of an object and of second image data of the object, wherein the partial registration is part of a registration procedure in which a user manually performs a further part of the registration and wherein
a) the first image data are generated by an ultrasound detector and/or have been generated by an ultrasound detector,
b) the second image data are three-dimensional image data,
c) a two-dimensional slice of the second image data is displayed on a display device (6), wherein the slice is located in a cut plane of the second image data, and wherein the slice comprises the surface of the object,
d) the first image data are displayed on the display device (6),
**characterized in that**
e) in response to a selection of the slice by the user, a straight line (35a) in the slice is automatically computed, wherein the straight line (35a) extends in a viewing direction of the ultrasound detector,
f) a surface point (36a), which is a point on the surface of the object, is automatically identified in the slice by determining a point of the straight line (35a) as the surface point, where the straight line (35a) intersects the surface of the object In the slice,
g) reference information defining a second point (36b) in the first image data, wherein the second point (36b) in the first image data is located on a surface of the object when the ultrasound detector generates the first image data of the object, are used,
h) in response to an adjustment of the orientation of the ultrasound detector (1) and/or to a selection of an appropriate image from the second image data made by the user, so that an orientation of the first image and of the second image on the display device (6) is aligned, the reference information is used and the partial registration is automatically finished by aligning the first and second image data in a manner in which the second point (36b) and the surface point (36a) are displayed at positions having the same coordinate with respect to only one coordinate axis of the display device, wherein the coordinate axis extends In the direction of the straight line (35a).

2. The method of claim 1, wherein the reference information is generated by the ultrasound detector.

3. The method of the preceding claim, wherein the direction of the straight line (35a) is fixed for a given cut plane.

4. The method of one of the preceding claims, wherein the slice and the second image data are superimposed on the display device (6), wherein the point (36b) in the first image data and the surface point (36a) are located at the same position of the display device.

5. The method of one of the preceding claims, wherein the surface point (36a) is identified comprising the following step: comparing data values of data points with a threshold value.

6. The method of one of the preceding claims, wherein the surface point (36a) is identified comprising the following step: evaluating data values of neighbouring data points and identifying a location where the local partial derivative of the data values matches a threshold value or matches or exceeds a threshold value.

7. An apparatus (5; 46) for automatically performing a partial registration of first image data of an object and of second image data of the object, wherein the partial registration is part of a registration procedure in which a user manually performs a further part of the registration , the apparatus comprising:
• an interface (9) adapted to receive first image data, wherein the first image data are generated by an ultrasound detector and/or have been generated by an ultrasound detector;
• an interface adapted to receive second image data, wherein the second image data are three-dimensional image data, and/or a data storage (4) for storing the second image data;
• a combination device (5) adapted to combine the first and the second image data to display on a display device (6) the first image data and a slice of the second image data, wherein the slice is located in a cut plane of the second image data, and wherein the slice comprises the surface of the object;
**characterized in that**
• the apparatus is adapted, in response to a selection of the slice by the user, to automatically compute a straight line (35a) in the slice, wherein the straight line (35a) extends in a viewing direction of the ultrasound detector,
• the apparatus comprises an identification device (41) adapted to identify at least one surface point (36a) of the object and to automatically identify a surface point (36a), which is a point on the surface of the object, in the slice by determining a point of the straight line (35a) as the surface point, where the straight line (35a) intersects the surface of the object in the slice, wherein the identification device is connected to the interface adapted to receive the second image data and/or is connected to the data storage (4);
• the apparatus comprises an interface (7) adapted to receive reference information defining a second point (36b) in the first data is located on a surface of the object when the ultrasound detector generates the first image data of the object;
• the apparatus is adapted, in response to an adjustment of the orientation of the ultrasound detector (1) and/or to a selection of an appropriate image from the second image data made by the user, so that an orientation of the first image and of the second image on the display device (6) is aligned, to use the reference information and to automatically finish the partial registration by aligning the first and second image data in a manner in which the second point (36b) and the surface point (36a) are displayed at positions having the same coordinate with respect to only one coordinate axis of the display device, wherein the coordinate axis extends in the direction of the straight line (35a).

8. An arrangement comprising the apparatus (5) of the preceding claim and further comprising an ultrasound detector (1), wherein the ultrasound detector (1) and the interface (7) adapted to receive the reference information are directly connected via a data connection (12) which is adapted to transfer geometry data (geometry data connection), wherein the geometry data comprise one or more than one of the following types of information:
• information concerning at least one spatial dimension of an image unit of the first image data;
• information concerning an image position of at least a part of an image, which is represented by the first image data, relative to a reference point of the ultrasound detector or relative to a reference point or reference object in the ultrasound image;
• information concerning an orientation of the ultrasound Image relative to a reference point or a reference object of the ultrasound detector,
• information concerning a region or an area, which is actually covered by an ultrasound image that is represented by the first image data; and
• information concerning a detector position of the ultrasound detector
and wherein the arrangement is adapted so that the ultrasound detector (1) generates the reference information and so that the reference information is transferred via the geometry data connection (12) to the apparatus (5).

9. A computer program product loadable into an internal memory of a computer or of a computer network, comprising program code portions for performing steps c) to h) of the method of claim 1 using data according to items a) and b) of claim 1 or code portions for performing this method with additional features defined in any one of claims 2 to 6 when the product is executed on the computer.

10. A storage medium, wherein a data structure is stored on the storage medium and wherein the data structure is adapted to perform steps c) to h) of the method of claim 1 using data according to items a) and b) of claim 1 or is adapted to perform this method with the additional features defined in any one of claims 2 to 6, after having been loaded into a main and/or working storage of a computer or of a computer network.

## Patentansprüche

1. Verfahren zum automatischen Durchführen einer teilweisen Registrierung von ersten Bilddaten eines Objekts und von zweiten Bilddaten des Objekts, wobei die teilweise Registrierung Teil einer Registrierungsprozedur ist, in der ein Benutzer einen weiteren Teil der Registrierung manuell durchführt, und wobei
a) die ersten Bilddaten von einem Ultraschalldetektor erzeugt werden und/oder von einem Ultraschalldetektor erzeugt worden sind,
b) die zweiten Bilddaten dreidimensionale Bilddaten sind,
c) ein zweidimensionaler Schnitt der zweiten Bilddaten auf einer Anzeigevorrichtung (6) angezeigt wird, wobei der Schnitt in einer Schnittebene der zweiten Bilddaten angeordnet ist und wobei der Schnitt die Oberfläche des Objekts aufweist,
d) die ersten Bilddaten auf der Anzeigevorrichtung (6) angezeigt werden,
**dadurch gekennzeichnet, dass**
e) in Reaktion auf eine Auswahl des Schnitts durch den Benutzer eine gerade Linie (35a) in dem Schnitt automatisch berechnet wird, wobei die gerade Linie (35a) sich in einer Blickrichtung des Ultraschalldetektors erstreckt,
f) ein Oberflächenpunkt (36a), der ein Punkt auf der Oberfläche des Objekts ist, automatisch in dem Schnitt identifiziert wird, indem ein Punkt auf der geraden Linie (35a) als der Oberflächenpunkt bestimmt wird, wo die gerade Linie (35a) die Oberfläche des Objekts in dem Schnitt schneidet,
g) Referenzinformation verwendet wird, die einen zweiten Punkt (36b) in den ersten Bilddaten definiert, wobei der zweite Punkt (36b) in den ersten Bilddaten auf einer Oberfläche des Objekts angeordnet ist, wenn der Ultraschalldetektor die ersten Bilddaten des Objekts erzeugt,
h) in Reaktion auf eine Anpassung der Orientierung des Ultraschalldetektors (1) und/oder auf eine Auswahl eines geeigneten Bildes aus den zweiten Bilddaten durch den Benutzer, so dass eine Orientierung des ersten Bildes und des zweiten Bildes auf der Anzeigevorrichtung (6) zueinander ausgerichtet sind, wird die Referenzinformation verwendet und wird die teilweise Registrierung automatisch beendet, indem die ersten und zweiten Bilddaten in einer Weise ausgerichtet werden, in der der zweite Punkt (36b) und der Oberflächenpunkt (36a) an Positionen angezeigt werden, die bezüglich lediglich einer Koordinatenachse der Anzeigevorrichtung dieselbe Koordinate haben, wobei die Koordinatenachse sich in der Richtung der geraden Linie (35a) erstreckt.

2. Verfahren nach Anspruch 1, wobei die Referenzinformation von dem Ultraschalldetektor erzeugt wird.

3. Verfahren nach dem vorgehenden Anspruch, wobei die Richtung der geraden Linie (35a) für eine gegebene Schnittebene fest ist.

4. Verfahren nach einem der vorhergehenden Ansprüche, wobei der Schnitt und die zweiten Bilddaten auf der Anzeigevorrichtung (6) überlagert werden, wobei der Punkt (36b) in den ersten Bilddaten und der Oberflächenpunkt (36a) an derselben Position der Anzeigevorrichtung angeordnet sind.

5. Verfahren nach einem der vorhergehenden Ansprüche, wobei der Oberflächenpunkt (36a) identifiziert wird, wobei der folgende Schritt ausgeführt wird: Vergleichen von Datenwerten von Datenpunkten mit einem Grenzwert.

6. Verfahren nach einem der vorhergehenden Ansprüche, wobei der Oberflächenpunkt (36a) identifiziert wird, wobei der folgende Schritt ausgeführt wird: Auswerten von Datenwerten von benachbarten Datenpunkten und Identifizieren eines Ortes, wo die lokale partielle Ableitung der Datenwerte mit einem Grenzwert zusammenfällt oder mit einem Grenzwert zusammenfällt oder diesen überschreitet.

7. Vorrichtung (5; 46) zum automatischen Durchführen einer teilweisen Registrierung von ersten Bilddaten eines Objekts und von zweiten Bilddaten des Objekts, wobei die teilweise Registrierung Teil einer Registrierungsprozedur ist, in der ein Benutzer einen weiteren Teil der Registrierung manuell durchführt, wobei die Vorrichtung aufweist:
. eine Schnittstelle (9), die ausgestaltet ist, erste Bilddaten zu empfangen, wobei die ersten Bilddaten von einem Ultraschalldetektor erzeugt werden und/oder von einem Ultraschalldetektor erzeugt worden sind;
. eine Schnittstelle, die ausgestaltet ist, zweite Bilddaten zu empfangen, wobei die zweiten Bilddaten dreidimensionale Bilddaten sind, und/oder einen Datenspeicher (4) zum Speichern der zweiten Bilddaten;
. eine Kombinationsvorrichtung (5), die ausgestaltet ist, die ersten und zweiten Bilddaten zu kombinieren, um die ersten Bilddaten und einen Schnitt der zweiten Bilddaten auf einer Anzeigevorrichtung (6) anzuzeigen, wobei der Schnitt in einer Schnittebene der zweiten Bilddaten angeordnet ist und wobei der Schnitt die Oberfläche des Objekts aufweist;
**dadurch gekennzeichnet, dass**
. die Vorrichtung ausgestaltet ist, in Reaktion auf eine Auswahl des Schnitts durch den Benutzer automatisch eine gerade Linie (35a) in dem Schnitt zu berechnen, wobei die gerade Linie (35a) sich in einer Blickrichtung des Ultraschalldetektors erstreckt,
. die Vorrichtung eine Identifizierungseinrichtung (41) aufweist, die ausgestaltet ist, zumindest einen Oberflächenpunkt (36a) des Objekts zu identifizieren und automatisch einen Oberflächenpunkt (36a) zu identifizieren, der ein Punkt auf der Oberfläche des Objekts ist, in dem Schnitt, indem ein Punkt auf der geraden Linie (35a) als der Oberflächenpunkt bestimmt wird, wo die gerade Linie (35a) die Oberfläche des Objekts in dem Schnitt schneidet, wobei die Identifizierungseinrichtung mit der Schnittstelle verbunden ist, die ausgestaltet ist, die zweiten Bilddaten zu empfangen, und/oder mit dem Datenspeicher (4) verbunden ist;
. die Vorrichtung eine Schnittstelle (7) aufweist, die ausgestaltet ist, Referenzinformation zu empfangen, die einen zweiten Punkt (36b) in den ersten Bilddaten definiert, wobei der zweite Punkt (36b) in den ersten Bilddaten auf einer Oberfläche des Objekts angeordnet ist, wenn der Ultraschalldetektor die ersten Bilddaten des Objekts erzeugt;
. die Vorrichtung ausgestaltet ist, in Reaktion auf eine Anpassung der Orientierung des Ultraschalldetektors (1) und/oder auf eine Auswahl eines geeigneten Bildes aus den zweiten Bilddaten durch den Benutzer, so dass eine Orientierung des ersten Bildes und des zweiten Bildes auf der Anzeigevorrichtung (6) zueinander ausgerichtet ist, die Referenzinformation zu verwenden und die teilweise Registrierung automatisch zu beenden, indem die ersten und zweiten Bilddaten in einer Weise ausgerichtet werden, in der der zweite Punkt (36b) und der Oberflächenpunkt (36a) an Positionen angezeigt werden, die in Bezug auf lediglich eine Koordinatenachse der Anzeigevorrichtung dieselbe Koordinate haben, wobei die Koordinatenachse sich in der Richtung der geraden Linie (35a) erstreckt.

8. Anordnung, die die Vorrichtung (5) des vorhergehenden Anspruchs aufweist und weiterhin einen Ultraschalldetektor (1) aufweist, wobei der Ultraschalldetektor (1) und die Schnittstelle (7), die ausgestaltet ist, die Referenzinformation zu empfangen, direkt über eine Datenverbindung (12) verbunden sind, die ausgestaltet ist, Geometriedaten zu übertragen (Geometriedaten-Verbindung), wobei die Geometriedaten einen oder mehr als einen der folgenden Typen von Informationen aufweisen:
. Informationen, die zumindest eine räumliche Dimension einer Bildeinheit der ersten Bilddaten betreffen;
. Informationen, die eine Bildposition von zumindest einem Teil eines Bildes betreffen, welches durch die ersten Bilddaten repräsentiert wird, relativ zu einem Referenzpunkt des Ultraschalldetektors oder relativ zu einem Referenzpunkt oder Referenzobjekt in dem Ultraschallbild;
. Informationen, die eine Orientierung des Ultraschallbildes relativ zu einem Referenzpunkt oder einem Referenzobjekt des Ultraschalldetektors betreffen;
. Informationen, die einen Bereich oder eine Fläche betreffen, der/die tatsächlich durch das Ultraschallbild, welches durch die ersten Bilddaten repräsentiert wird, abgedeckt ist; und
· Informationen, die eine Detektorposition des Ultraschalldetektors betreffen; und wobei die Anordnung so ausgestaltet ist, dass der Ultraschalldetektor (1) die Referenzinformation erzeugt und dass die Referenzinformation über eine Geometriedaten-Verbindung (12) zu der Vorrichtung (5) übertragen wird.

9. Computerprogrammprodukt, das in einen inneren Speicher eines Computers oder eines Computernetzes ladbar ist, aufweisend Programmcode-Abschnitte zum Ausführen der Schritte c) bis h) des Verfahrens nach Anspruch 1, unter Verwendung von Daten entsprechend der Punkte a) und b) nach Anspruch 1 oder Codeabschnitte zum Ausführen dieses Verfahrens mit zusätzlichen Merkmalen, die in einem der Ansprüche 2 bis 6 definiert sind, wenn das Produkt auf einem Computer ausgeführt wird.

10. Speichermedium, wobei eine Datenstruktur auf dem Speichermedium gespeichert ist und wobei die Datenstruktur ausgestaltet ist, die Schritte c) bis h) des Verfahrens nach Anspruch 1 auszuführen, unter Verwendung von Daten gemäß den Punkten a) und b) nach Anspruch 1, oder ausgestaltet ist, dieses Verfahren mit zusätzlichen Merkmalen auszuführen, die in einem der Ansprüche 2 bis 6 definiert sind, nachdem sie in einen Haupt- und/oder Arbeitsspeicher eines Computers oder eines Computernetzes geladen worden ist.

## Revendications

1. Procédé pour effectuer automatiquement un enregistrement partiel de premières données d'image d'un objet et de secondes données d'image de l'objet, dans lequel l'enregistrement partiel fait partie d'une procédure d'enregistrement dans laquelle un utilisateur effectue manuellement une autre partie de l'enregistrement et dans lequel
a) les premières données d'image sont générées par un détecteur d'ultrasons et/ou ont été générées par un détecteur d'ultrasons,
b) les secondes données d'image sont des données d'image tridimensionnelles,
c) une tranche bidimensionnelle des secondes données d'image est affichée sur un dispositif d'affichage (6), dans lequel la tranche est située dans un plan de coupe des secondes données d'image, et dans lequel la tranche comprend la surface de l'objet,
d) les premières données d'image sont affichées sur le dispositif d'affichage (6),
**caractérisé en ce que**
e) en réponse à une sélection de la tranche par l'utilisateur, une ligne droite (35a) dans la tranche est automatiquement calculée, dans lequel la ligne droite (35a) s'étend dans une direction de visualisation du détecteur d'ultrasons,
f) un point de surface (36a), qui est un point sur la surface de l'objet, est automatiquement identifié dans la tranche par la détermination d'un point de la ligne droite (35a) en tant que point de surface, où la ligne droite (35a) est en intersection avec la surface de l'objet dans la tranche,
g) des informations de référence définissant un second point (36b) dans les premières données d'image, dans lequel le second point (36b) dans les premières données d'image est situé sur une surface de l'objet lorsque le détecteur d'ultrasons génère les premières données d'image de l'objet, sont utilisées,
h) en réponse à un ajustement de l'orientation du détecteur d'ultrasons (1) et/ ou à une sélection d'une image appropriée à partir des secondes données d'image effectuée par l'utilisateur, de sorte qu'une orientation de la première image et de la seconde image sur le dispositif d'affichage (6) est alignée, les informations de référence sont utilisées et l'enregistrement partiel est automatiquement fini en alignant les premières données d'image et les secondes données d'image d'une manière dans laquelle le second point (36b) et le point de surface (36a) sont affichés à des positions ayant la même coordonnée par rapport à un seul axe de coordonnée du dispositif d'affichage, dans lequel l'axe de coordonnée s'étend dans la direction de la ligne droite (35a).

2. Procédé selon la revendication 1, dans lequel les informations de référence sont générées par le détecteur d'ultrasons.

3. Procédé selon la revendication précédente, dans lequel la direction de la ligne droite (35a) est fixe pour un plan de coupe donné.

4. Procédé selon l'une des revendications précédentes, dans lequel la tranche et les secondes données d'image sont superposées sur le dispositif d'affichage (6), dans lequel le point (36b) dans les premières données d'image et le point de surface (36a) sont situés à la même position du dispositif d'affichage.

5. Procédé selon l'une des revendications précédentes, dans lequel le point de surface (36a) est identifié en comprenant l'étape consistant à : comparer les valeurs de données de points de données à une valeur de seuil.

6. Procédé selon l'une des revendications précédentes, dans lequel le point de surface (36a) est identifié en comprenant l'étape consistant à : évaluer les valeurs de données de points de données voisins et identifier un emplacement où la dérivée partielle locale des valeurs de données correspond à une valeur de seuil ou correspond à ou dépasse une valeur de seuil.

7. Appareil (5 ; 46) pour effectuer automatiquement un enregistrement partiel de premières données d'image d'un objet et de secondes données d'image de l'objet, dans lequel l'enregistrement partiel fait partie d'une procédure d'enregistrement dans laquelle un utilisateur effectue manuellement une autre partie de l'enregistrement, l'appareil comprenant :
- une interface (9) adaptée pour recevoir des premières données d'image, dans lequel les premières données d'image sont générées par un détecteur d'ultrasons et/ ou ont été générées par un détecteur d'ultrasons ;
- une interface adaptée pour recevoir des secondes données d'image, dans lequel les secondes données d'image sont des données d'image tridimensionnelles, et/ ou un stockage de données (4) pour stocker les secondes données d'image ;
- un dispositif de combinaison (5) adapté pour combiner les premières données d'image et les secondes données d'image pour afficher sur un dispositif d'affichage (6) les premières données d'image et une tranche des secondes données d'image, dans lequel la tranche est située dans un plan de coupe des secondes données d'image, et dans lequel la tranche comprend la surface de l'objet ;
**caractérisé en ce que**
- l'appareil est adapté, en réponse à une sélection de la tranche par l'utilisateur, pour automatiquement calculer une ligne droite (35a) dans la tranche, dans lequel la ligne droite (35a) s'étend dans une direction de visualisation du détecteur d'ultrasons,
- l'appareil comprend un dispositif d'identification (41) adapté pour identifier au moins un point de surface (36a) de l'objet et pour automatiquement identifier un point de surface (36a), qui est un point sur la surface de l'objet, dans la tranche par la détermination d'un point de la ligne droite (35a) en tant que point de surface, où la ligne droite (35a) est en intersection avec la surface de l'objet dans la tranche, dans lequel le dispositif d'identification est connecté à l'interface adaptée pour recevoir les secondes données d'image et/ ou est connecté au stockage de données (4) ;
- l'appareil comprend une interface (7) adaptée pour recevoir des informations de référence définissant un second point (36b) dans les premières données d'image, dans lequel le second point (36b) dans les premières données d'image est situé sur une surface de l'objet lorsque le détecteur d'ultrasons génère les premières données d'image de l'objet ;
- l'appareil est adapté, en réponse à un ajustement de l'orientation du détecteur d'ultrasons (1) et/ou à une sélection d'une image appropriée à partir des secondes données d'image effectuée par l'utilisateur, de sorte qu'une orientation de la première image et de la seconde image sur le dispositif d'affichage (6) est alignée, pour utiliser les informations de référence et finir automatiquement l'enregistrement partiel en alignant les premières données d'image et les secondes données d'image d'une manière dans laquelle le second point (36b) et le point de surface (36a) sont affichés à des positions ayant la même coordonnée par rapport à un seul axe de coordonnée du dispositif d'affichage, dans lequel l'axe de coordonnée s'étend dans la direction de la ligne droite (35a).

8. Agencement comprenant l'appareil (5) selon la revendication précédente et comprenant en outre un détecteur d'ultrasons (1), dans lequel le détecteur d'ultrasons (1) et l'interface (7) adaptée pour recevoir les informations de référence sont directement connectés par l'intermédiaire d'une connexion de données (12) qui est adaptée pour transférer des données de géométrie (connexion de données de géométrie), dans lequel les données de géométrie comprennent un ou plusieurs des types suivants d'informations :
- des informations concernant au moins une dimension spatiale d'une unité d'image des premières données d'image ;
- des informations concernant une position d'image d'au moins une partie d'une image, qui est représentée par les premières données d'image, par rapport à un point de référence du détecteur d'ultrasons ou par rapport à un point de référence ou un objet de référence dans l'image d'ultrasons ;
- des informations concernant une orientation de l'image d'ultrasons par rapport à un point de référence ou un objet de référence du détecteur d'ultrasons ;
- des informations concernant une région ou une zone, qui est en fait recouverte d'une image d'ultrasons qui est représentée par les premières données d'image ; et
- des informations concernant une position de détecteur du détecteur d'ultrasons
et dans lequel l'agencement est adapté de manière à ce que le détecteur d'ultrasons (1) génère les informations de référence et de manière à ce que les informations de référence soient transférées par l'intermédiaire de la connexion de données de géométrie (12) à l'appareil (5).

9. Produit de programme d'ordinateur chargeable dans une mémoire interne d'un ordinateur ou d'un réseau d'ordinateurs, comprenant des portions de code de programme pour effectuer les étapes c) à h) du procédé de la revendication 1 en utilisant des données selon les éléments a) et b) de la revendication 1 ou des portions de code pour effectuer ce procédé avec des caractéristiques supplémentaires définies dans l'une quelconque des revendications 2 à 6 lorsque le produit est exécuté sur l'ordinateur.

10. Support de stockage, dans lequel une structure de données est stockée sur le support de stockage et dans lequel la structure de données est adaptée pour effectuer les étapes c) à h) du procédé de la revendication 1 en utilisant des données selon les éléments a) et b) de la revendication 1 ou est adapté pour effectuer ce procédé avec les caractéristiques supplémentaires définies dans l'une quelconque des revendications 2 à 6, après avoir été chargé dans un stockage principal et/ou de fonctionnement d'un ordinateur ou d'un réseau d'ordinateurs.
